Europäisches Patentamt

European Patent Office

Office européen des brevets

(19)

(11) Veröffentlichungsnummer : **0 032 525**
**B1**

(12) **EUROPÄISCHE PATENTSCHRIFT**

(45) Veröffentlichungstag der Patentschrift :
13.07.83

(21) Anmeldenummer : 80106242.3

(22) Anmeldetag : 15.10.80

(51) Int. Cl.³ : **B 01 J 23/94**, C 07 C 51/12,
C 07 C 51/48

(54) Verfahren zur hydrierenden Aufarbeitung der bei der Hydrocarboxylierung eingesetzten kobalthaltigen Katalysatoren.

(30) Priorität : **12.12.79 DE 2949878**

(43) Veröffentlichungstag der Anmeldung :
**29.07.81 Patentblatt 81/30**

(45) Bekanntmachung des Hinweises auf die Patenterteilung : **13.07.83 Patentblatt 83/28**

(84) Benannte Vertragsstaaten :
**BE FR GB IT NL**

(56) Entgegenhaltungen :
DE A 2 447 068
DE A 2 912 489
DE C 929 250
FR A 1 436 123
US A 2 529 236
US A 2 542 558
US A 3 507 891

(73) Patentinhaber : **CHEMISCHE WERKE HÜLS AG**
**Postfach 1320**
**D-4370 Marl 1 (DE)**

(72) Erfinder : **Hofmann, Peter, Dr.**
**Lipper Weg 193**
**D-4370 Marl (DE)**

# Verfahren zur hydrierenden Aufarbeitung der bei der Hydrocarboxylierung eingesetzten kobalthaltigen Katalysatoren

Es ist bekannt, daß man durch Umsetzung von Olefinen mit Kohlenmonoxid und einer H-aciden Komponente, wie Wasser oder Alkanol, in Gegenwart eines Katalysators, der ein Metall der 8. Nebengruppe des Periodischen Systems der Elemente und gegebenenfalls einen Promotor enthält, Fettsäuren bzw. die entsprechenden Fettsäurederivate herstellen kann (J. Falbe, Synthesen mit Kohlenmonoxid, Springer-Verlag, Berlin, Heidelberg, New York, 1967).

Als besonders bevorzugte Variante dieser als Hydrocarboxylierung bezeichneten Reaktion hat sich die Umsetzung in Gegenwart von kobalthaltigen Katalysatoren erwiesen. Eine besonders bevorzugte Ausführungsform besteht darin, daß man zusätzlich einen Promotor, und zwar insbesondere Pyridin oder ein nicht-orthosubstituiertes Alkylpyridin zusetzt.

Ein wesentliches Problem dieser homogenen katalysierten Reaktion ist die Rückgewinnung des relativ teuren Kobalts aus dem Reaktionsgemisch in einer Form, die seinen Wiedereinsatz als Katalysator gestattet.

Ein in der DE-AS 21 59 139 beschriebenes Verfahren löst dieses Problem dadurch, daß die Umsetzung des Olefins mit Kohlenmonoxid in Gegenwart eines Überschusses an Alkanol und Paraffin durchgeführt wird bzw. Paraffin nach beendeter Umsetzung zugegeben wird. Auf diese Weise bildet sich ein zweiphasiges Gemisch. Die untere Phase, die überwiegend aus Alkanol und Promotor besteht, enthält maximal ca. 97 % des als Katalysator eingesetzten Kobalts. Die obere paraffinische Phase wird im wesentlichen aus unumgesetztem Olefin und Reaktionsprodukten gebildet.

Die untere, den Katalysator noch in aktiver Form enthaltende Phase kann erneut in die Reaktion eingesetzt werden. Der sich hieraus ergebende Vorteil wird jedoch durch den Verlust von ca. 3 % des eingesetzten Kobalts mehr als aufgewogen. Ein Hydrocarboxylierungsverfahren kann nämlich nur dann als wirtschaftlich zufriedenstellend betrachtet werden, wenn auch das in der paraffinischen Phase enthaltende Kobalt zurückgewonnen wird. Angesichts des für das Verfahren erforderlichen Alkanolüberschusses und Paraffinzusatzes würde sich eine solche Aufarbeitung allerdings sehr aufwendig gestalten.

Ein weiteres Verfahren zur Rückgewinnung des Kobalts ist in der US-PS 3 507 891 beschrieben. Das Verfahren ist dadurch gekennzeichnet, daß das Kobalt zusammen mit dem Sumpf der destillativen Aufarbeitung des Reaktionsgemisches wiedergewonnen wird.

Wird dabei das Reaktionsgemisch vor der destillativen Aufarbeitung einer oxidativen Behandlung, z. B. mit Luft, unterzogen, so wird der Katalysator in einer Form zurückgewonnen, aus der sich erst wieder unter den Bedingungen der Hydrocarboxylierung die aktive Katalysatorspecies zurückbildet. Nur beim Einsatz von Alkylpyridinen als Promotoren soll auf eine oxidative Behandlung des Reaktionsgemisches verzichtet werden können. In Gegenwart dieser Promotoren sollen sich nämlich unter den Destillationsbedingungen thermostabile Komplexe bilden, die ihre Aktivität beibehalten.

Das Verfahren gemäß der US-PS 3 507 891 bietet zwar eine Möglichkeit zur weitgehend vollständigen Rückgewinnung des eingesetzten Kobalts, es zeigt aber ebenso wenig wie das Verfahren gemäß der DE-AS 21 59 139 einen Weg zur Abtrennung von hochsiedenden Substanzen und anderen störenden Verunreinigungen, die sich unvermeidlich bei jeder Hydrocarboxylierung als Nebenprodukt bilden.

Aufgabe der vorliegenden Erfindung war es daher, ein möglichst einfaches und weitgehend verlustfreies Verfahren zur Aufarbeitung des bei der Hydrocarboxylierung eingesetzten kobalthaltigen Katalysators zu entwickeln, das gleichzeitig die Abtrennung von hochsiedenden und unerwünschten Verunreinigungen gestattet.

Diese Aufgabe wurde dadurch gelöst, daß man das Reaktionsgemisch nach einer oxidativen Behandlung hydriert, das dabei anfallende metallische Kobalt abtrennt und es nach Behandlung mit einer Säure in Form einer mindestens in einem der Reaktanten löslichen Verbindung in das Verfahren zurückführt.

Das erfindungsgemäße Verfahren kann im Prinzip bei allen Hydrocarboxylierungsreaktionen, die in Gegenwart eines kobalthaltigen Katalysators durchgeführt werden, angewandt werden (z. B. Verfahren gemäß US-PS 3 507 891 und deutscher Patentanmeldung P 29 12 489 8). So ist vor allem die Wahl des eingesetzten Olefins unkritisch, d. h. es können sowohl geradkettige oder verzweigte $\alpha$-Olefine als auch Olefine mit innenständiger Doppelbindung eingesetzt werden. Aber auch Olefine mit mehr als einer Doppelbindung und solche mit Substituenten, z. B. Aryl-, Cyano-, Carboximethyl- und Hydroxylgruppen, sind geeignet.

Im allgemeinen werden Olefine mit 2 bis 40, vorzugsweise mit 4 bis 20 Kohlenstoffatomen, eingesetzt, die nach bekannten Verfahren des Standes der Technik erhalten werden können. So können z. B. $\alpha$-Olefine durch die Aufbaureaktion von Ethylen nach Ziegler oder durch Wachscracken, Olefine mit innenständiger Doppelbindung durch Dehydrierung oder Chlorierung und anschließender Dehydrochlorierung von Paraffinen gewonnen werden.

Bei dem zuletzt genannten Verfahren werden in der Regel Paraffinschnitte, d. h. Gemische unterschiedlicher C-Zahl eingesetzt, so daß auch die erhaltenen Olefine keine einheitliche C-Zahl aufweisen. Außerdem kommen in diesen Olefin-Gemischen natürlich alle denkbaren isomeren Formen vor.

Neben den reinen — gegebenenfalls substituierten — Olefinen können auch solche mit einem Gehalt an Paraffinen eingesetzt werden. Der Paraffingehalt rührt daher, daß bei der Olefinherstellung keine vollständigen Umsätze erreicht und die nicht umgesetzten Paraffine nicht oder nicht vollständig abgetrennt werden.

Nicht nur das eingesetzte Olefin, sondern auch die H-acide Verbindung, die mit dem Olefin und Kohlenmonoxid umgesetzt wird, ist für das erfindungsgemäße Verfahren unkritisch. Sowohl Wasser als auch Alkanole mit 1 bis 20, vorzugsweise 1 bis 4 Kohlenstoffatomen, können eingesetzt werden.

Weiterhin ist es unwesentlich, welche Kobaltverbindung bei der Hydrocarboxylierung verwendet wird. Carbonyle des Kobalts sind ebenso geeignet wie carbonsaure Kobaltsalze bzw. Salze des Kobalts mit anorganischen Säuren. Vorzugsweise kommen solche carbonsauren Kobaltsalze zum Einsatz, deren Anionen in Form der entsprechenden Carbonsäuren bzw. Carbonsäureester bei der Hydrocarboxylierung gebildet werden.

Neben der Kobaltverbindung wird ein sogenannter Promotor eingesetzt. Hierfür kommen Pyridin, alle nicht orthosubstituierten Alkylpyridine und N-Methylpyrrolidon infrage.

Schließlich sind die Reaktionsbedingungen, unter denen die Hydrocarboxylierung durchgeführt wird, für das erfindungsgemäße Verfahren nicht von Bedeutung. Im allgemeinen werden Hydrocarboxylierungsverfahren bei Temperaturen von 80 bis 300, vorzugsweise 150 bis 220 °C, und Kohlenmonoxiddrucken von 10 bis 800, vorzugsweise 100 bis 300 bar, durchgeführt.

Verfahrenskritisch für das vorliegende Verfahren ist jedoch die oxidative Behandlung des Reaktionsgemisches vor der Rückgewinnung des Kobalts mit Sauerstoff oder einem sauerstoffhaltigen Gas, vorzugsweise Luft, bei Temperaturen von 20 bis 150, vorzugsweise 70 bis 120 °C. Diese Behandlung, die bereits in der US-PS 3 507 891, Spalte 4, Zeilen 21 bis 43, und in der nicht zum Stand der Technik gehörenden deutschen Patentanmeldung P 29 12 489 8, Seite 5, Absatz 2, beschrieben ist, wird solange durchgeführt, bis die bei der folgenden destillativen Aufarbeitung zur Abscheidung von metallischem Kobalt führenden Kobaltverbindungen oxidativ zerstört sind.

Bei der sich anschließenden destillativen Aufarbeitung werden die flüchtigen Anteile des Reaktionsgemisches entweder in einem Schritt oder stufenweise bei Sumpftemperaturen bis maximal 350 °C abgetrennt. Der Kobaltgehalt des hierbei anfallenden Destillationssumpfes soll 2 bis 30, vorzugsweise 4 bis 15 Gewichtsprozent, betragen.

Der kobalthaltige Rückstand kann entweder in seiner Gesamtheit oder teilweise erfindungsgemäß aufgearbeitet werden. Wählt man die Aufarbeitung nur eines Teils des kobalthaltigen Rückstandes, so richtet sich der Anteil des aufzuarbeitenden kobalthaltigen Rückstandes nach dem für die Hydrocarboxylierung angestrebten Niveau katalytischer Aktivität, der tolerierbaren Menge an Ballaststoffen, wie Hochsiedern, und dem für die Aufarbeitung erforderlichen Aufwand.

Erfindungsgemäß geht man so vor, daß man den aufzuarbeitenden Destillationssumpf bei erhöhtem Druck und bei erhöhter Temperatur einer hydrierenden Behandlung unterwirft, die überraschenderweise ohne Zusatz eines speziellen Hydrierkatalysators durchgeführt werden kann. Im allgemeinen arbeitet man bei Temperaturen von 20 bis 300 °C, vorzugsweise von 140 bis 220 °C. Der zur Hydrierung erforderliche Wasserstoffdruck beträgt im allgemeinen 50 bis 500 bar, vorzugsweise 150 bis 300 bar.

Obwohl die Hydrierung des kobalthaltigen Sumpfes auch in Abwesenheit eines Lösemittels durchgeführt werden kann, ist es dennoch zweckmäßig, ein solches zu verwenden. Als Lösemittel können z. B. Alkanole, vorzugsweise Methanol, Paraffine, vorzugsweise solche mit 5 bis 8 Kohlenstoffatomen, z. B. $C_5$-Schnitt, Hexan oder Cyclohexan, oder Carbonsäuren, vorzugsweise Essigsäure oder Propionsäure, eingesetzt werden. Die Gewichtsmenge des Lösemittels kann das 0,1- bis 10fache des Destillationssumpfes betragen.

Nach einer Hydrierdauer von bis zu 10 Stunden, vorzugsweise bis zu 5 Stunden, wird das anfallende Reaktionsgemisch z. B. durch Filtration in einen aus metallischem Kobalt bestehenden Rückstand und eine organische Phase zerlegt. Dabei ist es zweckmäßig, unter einer Inertgasatmosphäre von z. B. Stickstoff oder Argon zu arbeiten, da das Kobalt in sehr feinverteilter und daher pyrophorer Form anfallen kann.

Die bei der Trennung anfallende organische Phase kann destillativ aufgearbeitet werden. Dabei kann das gegebenenfalls bei der Hydrierung eingesetzte Lösemittel zurückgewonnen und die übrigen Produkte gegebenenfalls einer sinnvollen Verwertung zugeführt werden.

Da man das metallische Kobalt in der Regel nicht als solches in einen Hydrocarboxylierungsprozeß zurückführen kann, muß es durch Behandlung mit einer anorganischen Säure bzw. einer Carbonsäure in ein Salz überführt werden. Als besonders vorteilhaft hat sich eine Umsetzung des metallischen Kobalts mit $C_1$- bis $C_4$-Carbonsäuren, vorzugsweise Essigsäure und/oder Propionsäure, erwiesen. Um den Auflösungsprozeß des metallischen Kobalts in organischen Säuren zu verkürzen, ist es zweckmäßig, bei erhöhter Temperatur, z. B. unter Rückfluß, bei gleichzeitigem Durchleiten eines sauerstoffhaltigen Gases, z. B. Luft, und in Anwesenheit von Wasser zu arbeiten.

Ist das anfallende Kobaltsalz in den zur Hydrocarboxylierung eingesetzten Reaktanten nicht oder nicht genügend löslich, so ist als letzter Schritt eine Umwandlung erforderlich. Diese besteht in einer Umsetzung mit einer solchen Carbonsäure, deren Kobaltsalz dann in mindestens einem der Reaktanten löslich ist. Zum Beispiel kann Kobaltacetat, das sich nicht in höheren Alkanolen, Olefinen und den eingesetzten Promotoren löst, mit Hilfe von z. B. 2-Ethylhexansäure in das sogenannte Kobaltoctoat überführt werden, das in Alkanolen mit einer C-Zahl von ≥ 2 löslich ist. Die bei der obengenannten Umsetzung freiwerdende Essigsäure kann destillativ abgetrennt und in den Aufarbeitungsprozeß zurückgeführt werden.

Wie bereits dargelegt, kann das erfindungsgemäße Verfahren bei allen Hydrocarboxylierungsprozessen mit Erfolg eingesetzt werden, bei denen ein kobalthaltiger Katalysator verwendet wird.

Alle Prozentangaben — auch in den nachfolgenden Beispielen, die das erfindungsgemäße Verfahren erläutern — sind, sofern nicht anders angegeben, Gewichtsprozente.

Beispiel 1

Hydrocarboxylierung :
In einem 5 l-VA-Rührautoklaven werden 2 016 g eines Gemisches aus 40 Mol.-% n-Undecen, 40 Mol.-% n-Dodecen und 20 Mol.-% n-Tridecen (Olefine liegen jeweils als statistisches Isomerengemisch mit einem α-Olefinanteil < 1 % vor), 800 g Methanol, 167 g eines Gemisches aus dodecan-, tridecan- und tetradecansaurem Kobalt (Kobaltgehalt 10 %) und 279 g γ-Picolin bei 180 °C mit CO mit einem $H_2$-Gehalt von 2 Vol.-% bei 200 bar Warmdruck umgesetzt. Nach 3 Stunden wird die Reaktion bei einem Olefinumsatz von 83 % abgebrochen.

Oxidative Behandlung des Reaktionsaustrags :
Der gesamte Reaktionsaustrag von 3 495 g wird in einer mit Raschigringen gefüllten Rieselsäule (Länge : 1 m ; Innendurchmesser : 2,5 cm) bei 80 °C im Gegenstrom mit 100 l Luft/h behandelt. Die Verweilzeit der von oben zulaufenden flüssigen Phase in der Rieselsäule beträgt 15 min.

Katalysatoraufarbeitung :
Aus dem so vorbehandelten Reaktionsaustrag werden durch stufenweise Destillation Methanol, γ-Picolin, unumgesetztes Olefin und das Gemisch aus Dodecan-, Tridecan- und Tetradecansäuremethylester abgetrennt. Nach dem Abtrennen der Esterfraktion verbleibt ein Rückstand von 205 g mit einem Kobaltgehalt von 8,15 %.

Der kobalthaltige Rückstand wird in 410 g n-Hexan aufgenommen und in einem 2 l-VA-Rührautoklaven 5 Stunden lang bei 180 °C und einem $H_2$-Warmdruck von 300 bar hydriert. Der Autoklavenaustrag wird unter einer $N_2$-Schutzgasatmosphäre filtriert, der Filterkuchen wird 3mal mit insgesamt 100 ml n-Hexan ausgewaschen. Der grauschwarze, pulvrige Filterkuchen wiegt 19,72 g und hat einen Kobaltgehalt von 83,5 %. Damit befinden sich 98,6 % des als Hydrocarboxylierungskatalysator eingesetzten Kobalts im Filterkuchen.

Aus dem Filtrat wird das Hexan durch Destillation zurückgewonnen. Der kobalthaltige Filterkuchen wird in einem Gemisch aus 100 g Essigsäure und 100 g Wasser 2 Stunden unter Rückfluß gekocht. Durch das siedende Gemisch werden 30 l Luft/h geleitet. Die so erhaltene violette Lösung wird am Rotationsverdampfer bei Wasserstrahlvakuum und einer Badtemperatur von 55 °C zur Trockne eingeengt. Man erhält 64,1 g eines violetten, kristallinen Feststoffs mit einem Kobaltgehalt von 25,7 %.

Wiedereinsatz des zurückgewonnenen Kobalts als Hydrocarboxylierungskatalysator :
Die zu Beginn des Beispiels 1 beschriebene Hydrocarboxylierung wird unter den gleichen Bedingungen wiederholt, mit der Ausnahme, daß nun als Katalysator ein in Methanol gelöstes Gemisch aus 64,1 g des violetten, kristallinen Feststoffs und 920 mg Kobaltacetat (zum Ersatz der Kobaltverluste) eingesetzt wird. Die ebenfalls nach 3 Stunden abgebrochene Reaktion liefert das gleiche Ergebnis wie die zu Beginn beschriebene Hydrocarboxylierung.

Beispiel 2

Die in Beispiel 1 beschriebene Hydrocarboxylierung wird wiederholt, mit der Ausnahme, daß als Katalysator für die Hydrocarboxylierung 111,3 g Kobaltnaphthenat mit einem Kobaltgehalt von 15 % eingesetzt werden.

Die oxidative Behandlung des Reaktionsaustrags der Hydrocarboxylierung erfolgt unter den gleichen Bedingungen wie in Beispiel 1.

Als Sumpfprodukt einer stufenweisen destillativen Aufarbeitung des oxidativ vorbehandelten Reaktionsgemisches verbleiben 137,5 g Rückstand mit einem Kobaltgehalt von 12,13 %.

Dieser Rückstand wird unter den gleichen Bedingungen wie in Beispiel 1 hydriert. Der durch Filtration erhaltene kobalthaltige Filterkuchen wird wie in Beispiel 1 aufgelöst. Die erhaltene Lösung liefert nach dem Einengen zur Trockne 65,1 g eines violetten, kristallinen Feststoffs mit einem Kobaltgehalt von 25,4 %. Dies entspricht einer Kobaltrückgewinnung von 99,0 %.

Beispiel 3

Beispiel 1 wird wiederholt, mit der Ausnahme, daß nur 20 % des nach der destillativen Aufarbeitung des oxidativ behandelten Austrags der Hydrocarboxylierung verbleibenden kobalthaltigen Rückstands aufgearbeitet werden. Die Menge des zur Hydrierung als Lösemittel eingesetzten n-Hexans wird entsprechend reduziert. Als Endprodukt der Katalysatoraufarbeitung erhält man 12,9 g eines 25,6 % Kobalt enthaltenden violetten, kristallinen Feststoffs. Dies entspricht einer Kobaltrückgewinnung von 98,8 %, bezogen auf den aufgearbeiteten Anteil des kobalthaltigen Rückstands.

Der violette, kristalline Feststoff und 160 mg Kobaltacetat (zum Ersatz der Kobaltverluste) werden in Methanol gelöst und zusammen mit den 80 % nicht aufgearbeiteten Katalysatorrückständen als Hydrocarboxylierungskatalysator eingestzt. Dabei werden die Bedingungen der in Beispiel 1 zu Beginn beschriebenen Hydrocarboxylierung angewandt. Nach 3stündiger Reaktion beträgt der Olefinumsatz 83 %.

Beispiel 4

Beispiel 1 wird wiederholt, mit der Ausnahme, daß die hydrierende Katalysatoraufarbeitung bei 160 °C und 200 bar $H_2$-Warmdruck durchgeführt wird.

Der Grad der so erzielbaren Kobaltrückgewinnung liegt bei 98,5 %.

Beispiel 5

Beispiel 1 wird wiederholt, mit der Ausnahme, daß n-Hexan durch die gleiche Gewichtsmenge Methanol als Lösemittel für die hydrierende Katalysatoraufarbeitung verwendet wird.

Der Grad der so erzielbaren Kobaltrückgewinnung liegt bei 99,0 %.

Beispiel 6

Beispiel 5 wird wiederholt, mit der Ausnahme, daß nur die halbe Gewichtsmenge Methanol als Lösemittel für die hydrierende Katalysatoraufarbeitung verwendet wird.

Der Grad der so erzielbaren Kobaltrückgewinnung liegt bei 98,7 %.

Beispiel 7

Beispiel 1 wird wiederholt, jedoch wird anstelle von 800 g in der Hydrocarboxylierung eingesetzten Methanols die gleiche Gewichtsmenge an Ethanol verwendet.

Um den als Endprodukt der Katalysatoraufarbeitung verbleibenden violetten, kristallinen Feststoff, der 98,4 % des als Hydrocarboxylierungskatalysator eingesetzten Kobalts enthält, wieder als Lösung in einem der für die Hydrocarboxylierung verwendeten Reaktanten in die Reaktion zurückführen können, wird er in ein anderes carbonsaures Salz umgewandelt:

66,2 g des violetten, kristallinen Feststoffs, der sich in keinem der Reaktanten ausreichend löst, werden mit 147,3 g eines durch Hydrocarboxylierung nach Beispiel 1 hergestellten Gemisches aus Dodecan-, Tridecan- und Tetradecansäure versetzt und solange im Wasserstrahlvakuum erhitzt, bis keine Essigsäure und kein Wasser mehr abdestillieren. Das so erhaltene fettsaure Kobalt wird nach Ersatz der Kobaltverluste als ethanolische Lösung erneut als Katalysator für die Hydrocarboxylierung eingesetzt. Dabei werden die gleichen Bedingungen wie bei der zu Beginn des Beispiels 7 beschriebenen Hydrocarboxylierung eingehalten. Das Reaktionsergebnis beider Hydrocarboxylierungsansätze des Beispiels 7 unterscheidet sich nicht.

Beispiel 8

Beispiel 7 wird wiederholt, mit der Ausnahme, daß der durch Filtration erhaltene kobalthaltige Filterkuchen unter Verwendung der gleichen Gewichtsmenge Propionsäure anstelle von Essigsäure aufgearbeitet wird.

Als Endprodukt der Katalysatoraufarbeitung erhält man ein violettes, pastöses Produkt, das sich in Ethanol löst und 98,5 % des als Hydrocarboxylierungskatalysators eingesetzten Kobalts enthält.

Beispiel 9

Beispiel 1 wird wiederholt, mit der Ausnahme, daß anstelle des Gemisches von Olefinen mit innenständiger Doppelbindung 1 400 g α-Decen eingesetzt werden.

Der Grad der so erzielbaren Kobaltrückgewinnung beträgt 98,9 %.

**Ansprüche**

1. Verfahren zur Aufarbeitung eines kobalthaltigen Destillationssumpfes, der bei der destillativen Aufarbeitung eines durch Umsetzung von Olefinen mit Kohlenmonoxid und Wasser oder Alkanolen in Gegenwart eines kobalthaltigen Katalysators und eines Promotors erhaltenen und anschließend oxidativ behandelten Reaktionsgemisches anfällt, dadurch gekennzeichnet, daß man den als Destillationssumpf anfallenden kobalthaltigen Rückstand hydriert, das dabei anfallende metallische Kobalt abtrennt, es mit einer Säure umsetzt und das so erhaltene Kobaltsalz gegebenenfalls in ein anderes Kobaltsalz umwandelt.

2. Verfahren nach Anspruch 1, dadurch gekennzeichnet, daß man die Hydrierung in Gegenwart eines Lösemittels durchführt.

3. Verfahren nach den Ansprüchen 1 und 2, dadurch gekennzeichnet, daß man das metallische Kobalt bei erhöhter Temperatur und bei gleichzeitigem Durchleiten eines sauerstoffhaltigen Gases in einer wasserverdünnten Carbonsäure mit 1 bis 4 Kohlenstoffatomen auflöst.

4. Verfahren nach Anspruch 3, dadurch gekennzeichnet, daß man wäßrige Essigsäure verwendet.

**Claims**

1. A process for working-up a cobalt-containing distillation bottoms product which has arisen in the working-up by distillation of a reaction mixture obtained by reaction of an olefin with carbon monoxide and water or an alkanol in the presence of a cobalt-containing catalyst and a promoter and subsequently oxidised, characterised in that the cobalt-containing residue arising as distillation bottoms product is hydrogenated, the resulting metallic cobalt is separated and reacted with an acid and the cobalt salt formed is optionally converted into another cobalt salt.

2. A process according to claim 1, characterised in that the hydrogenation is carried out in the presence of a solvent.

3. A process according to claim 1 or 2, characterised in that the metallic cobalt is caused to dissolve in an aqueous $C_1$-$C_4$ carboxylic acid mixture by means of elevated temperature and simultaneous passage therethrough of an oxygen-containing gas.

4. A process according to claim 3, characterised in that aqueous acetic acid is used.

**Revendications**

1. Procédé de traitement d'une queue de distillation qui renferme du cobalt et se forme lorsqu'on traite par distillation un mélange réactionnel obtenu en faisant réagir des oléfines sur du monoxyde de carbone et de l'eau ou des alcanols en présence d'un catalyseur renfermant du cobalt et d'un promoteur, puis en traitant ensuite par oxydation,

ledit procédé étant caractérisé par le fait qu'on soumet à une hydrogénation le résidu renfermant du cobalt qui se forme en tant que queue de distillation, qu'on sépare ensuite le cobalt métallique qui se forme alors, qu'on le fait réagir sur un acide et qu'on transforme éventuellement le sel de cobalt ainsi obtenu en un autre sel de cobalt.

2. Procédé selon la revendication 1, caractérisé par le fait qu'on effectue l'hydrogénation en présence d'un solubilisant.

3. Procédé selon les revendications 1 et 2, caractérisé par le fait qu'en faisant passer simultanément un gaz oxygéné, on dissout le cobalt métallique, à température élevée, dans un acide carboxylique dilué à l'eau et comportant de 1 à 4 atomes de carbone.

4. Procédé selon la revendication 3, caractérisé par le fait qu'on utilise une solution aqueuse d'acide acétique.